Europäisches Patentamt

(19) European Patent Office

Office européen des brevets

(11) Veröffentlichungsnummer: **0 002 244**

**A1**

(12) # EUROPÄISCHE PATENTANMELDUNG

(21) Anmeldenummer: **78101463.4**

(22) Anmeldetag: **27.11.78**

(51) Int. Cl.²: **A 61 B 6/00**
**A 61 B 6/08**

(30) Priorität: **28.11.77 DE 2753047**

(43) Veröffentlichungstag der Anmeldung:
**13.06.79 Patentblatt 79/12**

(84) Benannte Vertragsstaaten:
**DE FR GB NL SE**

(71) Anmelder: **Novak, Robert, Dr.**
**c/o Siemens AG Postfach 261**
**D-8000 München 22(DE)**

(72) Erfinder: **Novak, Robert, Dr.**
**Amorvägen 8**
**S-18146 Lidingö(SE)**

(72) Erfinder: **Ytterfalk, Kjell**
**Cervins väg 62**
**S-16357 Spanga(SE)**

(74) Vertreter: **Mehl, Ernst, Dipl.-Ing.**
**Wittelsbacherplatz 2**
**D-8000 München 2(DE)**

(54) **Röntgenuntersuchungsgerät.**

(57) Die Erfindung bezieht sich auf ein Röntgenuntersuchungsgerät für die weibliche Brust, mit einer von einem Röntgengenerator gespeisten Röntgenstrahlenquelle (3) und einer Brustauflageplatte, an der eine Röntgenfilmkassette halterbar ist. Zwischen der Röntgenstrahlenquelle (3) und der Brustauflageplatte ist eine Kompressionsplatte (8) parallel zur Brustauflageplatte gehaltert, die relativ zur Brustauflageplatte zur Einstellung ihres Abstandes von der Brustauflageplatte einstellbar ist. Die Kompressionsplatte (8) weist einen Ausschnitt (12) solcher Größe auf, daß ein größerer Teil der Brust freigelassen wird. Am Rand des Ausschnitts ist eine auf einem Röntgenfilm abbildbare Markierung (13, 14) zum Aufsuchen eines bestimmten Punktes im Ausschnitt (12) angeordnet (Fig. 4).

EP 0 002 244 A1

·/· · ·

FIG 4

Dr.med. Robert Novak
Amorvägen 8
S-18146 Lidingö

Unser Zeichen
VPA 77 P 8911 EPC

### Röntgenuntersuchungsgerät

Die Erfindung betrifft ein Röntgenuntersuchungsgerät für die weibliche Brust, mit einer von einem Röntgengenerator gespeisten Röntgenstrahlenquelle und einer Brustauflageplatte, an der eine Röntgenfilmkassette halterbar ist, bei dem zwischen der Röntgenstrahlenquelle und der Brustauflageplatte eine Kompressionsplatte parallel zur Brustauflageplatte gehaltert ist, die relativ zur Brustauflageplatte zur Einstellung ihres Abstandes von der Brustauflageplatte einstellbar ist.

Ein Röntgenuntersuchungsgerät dieser Art ist aus dem Prospekt "Mammomat" der Firma Siemens bekannt. Die Kompressionsplatte ist dabei von der der Brustauflageplatte zugewandten Seite eines Kompressionstubusses gebildet. Wenn nach einer Brustaufnahme einer Patientin ein Tumor entdeckt wird, muß eine Gewebeprobe vom Tumor entnommen werden. Hierzu ist es bekannt, für eine Auf-

Lst 5 Kli / 24.7.1978

nahme die Brust zwischen der Auflageplatte und einer über ihre gesamte Fläche mit kleinen Löchern versehene Kompressionsplatte einzupressen (The American Journal of Roentgenology" 1974, Vol. 121, No. 1, Seiten 843 bis 845). Auf der Röntgenaufnahme sind die Löcher der Kompressionsplatte in Form von Ringen schwach aufgezeichnet. Wenn der Tumor auf der Aufnahme genau in einem solchen Ring liegt, kann der Arzt das entsprechende Loch auf der Platte aufsuchen und mittels einer Biopsienadel durch das Loch eine Punktur der Brust der sich noch im Röntgenuntersuchungsgerät befindlichen Patientin vornehmen, um so die Gewebeprobe zu entnehmen. Der Nachteil der Verwendung einer solchen Kompressionsplatte besteht darin, daß der Tumor in einer Aufnahme auch zwischen zwei Löchern liegen kann, so daß die Aufnahme wiederholt und hierzu die Platte gegenüber der Brust verschoben werden muß, was zeitraubend und für die Patientin belästigend ist. Ferner entstehen bei der Punktur häufig Blutungen, die auf die Kompressionsplatte gelangen können. Bei einer Weiterverwendung dieser Platte bei anderen Patientinnen können dadurch Krankheiten, wie z.B. Virushepatitis, übertragen werden. Ein weiterer Nachteil der bekannten, aus Plexiglas bestehenden Kompressionsplatte ist, daß sie die Bildqualität aufgrund von Sekundärstrahlung verschlechtert.

Der Erfindung liegt die Aufgabe zugrunde, ein Röntgenuntersuchungsgerät mit einer Kompressionsplatte der eingangs genannten Art zu schaffen, bei der die Bildqualität der Röntgenaufnahme nicht durch Streustrahlung in unerwünschter Weise verschlechtert wird und bei der in einem weiten Bereich ein Zugang zu beliebigen Stellen der Brust möglich ist, so daß in der Regel eine einzige Röntgenaufnahme zum Auffinden derjenigen Stelle in der Brust, an der Gewebe zu entnehmen ist, ausreicht. Ferner soll eine Verunreinigung der Kompressionsplatte durch Blutungen weitgehend ausgeschlossen sein.

Diese Aufgabe ist erfindungsgemäß dadurch gelöst, daß die Kompressionsplatte einen Ausschnitt solcher Größe aufweist, daß ein größerer Teil der Brust freigelassen wird und daß am Rand des Ausschnitts eine auf einem Röntgenfilm abbildbare Markierung zum Aufsuchen eines bestimmten Punktes im Ausschnitt angeordnet ist. Durch die Größe des Ausschnitts ist der aufzunehmende Teil der Brust freigelegt, so daß das Röntgenbild im diagnostisch wichtigen Bereich nicht durch Streustrahlung verschlechtert ist. Wird zunächst eine Brustaufnahme mit einer Kompressionsplatte ohne Aussparung gemacht, so kann anschließend die Brust für die zweite Aufnahme mit der Kompressionsplatte mit Aussparung so plaziert werden, daß die aufzusuchende Punkturstelle mit Sicherheit hinter der Aussparung liegt und durch die Aussparung zur Gewebeentnahme zugänglich ist. Die Punkturstelle kann dabei durch die ein Koordinationssystem bildende Markierung, die auch im Röntgenbild abgebildet wird, leicht gefunden werden. Ferner ist es möglich, das Blut von der Punkturstelle abzutrocknen, bevor es die Kompressionsplatte verunreinigt.

In einer vorteilhaften Ausbildung der Erfindung wird vorgeschlagen, daß die Kompressionsplatte im Randbereich ihres Ausschnitts auf ihrer der Brustauflageplatte zugewandten Seite in ihrer Dicke verstärkt ist. Dadurch ist erreicht, daß die Brusthaut in dem Ausschnitt gespannt ist, was eine genaue Punktur ermöglicht.

Eine besonders zweckmäßige Weiterbildung der Erfindung besteht darin, daß am Gehäuse der Röntgenstrahlenquelle eine Lichtvisieranordnung angebracht ist, deren zur Kompressionsplatte gerichtetes Lichtbündel durch Stellmittel einstellbar ist. Dadurch ist erreicht, daß die Punkturstelle durch eine Lichtmarke genau eingestellt werden kann.

Die Erfindung ist nachfolgend anhand eines in den Zeichnungen dargestellten Ausführungsbeispiels näher erläutert. Es zeigen:

    Fig. 1 ein Röntgenuntersuchungsgerät nach der Erfindung,

    Fig. 2 die Kompressionsplatte des Röntgenuntersuchungsgerätes nach Fig. 1 von oben,

    Fig. 3 einen Schnitt durch die Kompressionsplatte nach Fig. 2 nach der Linie III-III und

    Fig. 4 ein schematisch dargestelltes Lichtvisier für das Röntgenuntersuchungsgerät nach Fig. 1.

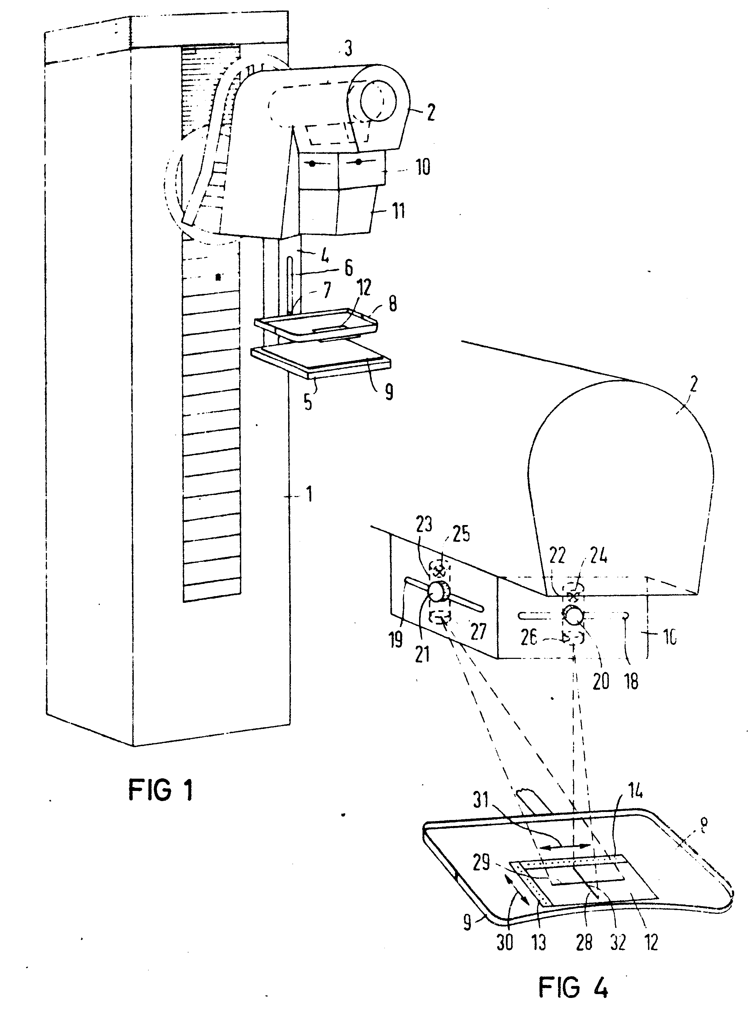

Die Fig. 1 zeigt ein Röntgenuntersuchungsgerät mit einem Schrank 1 und mit einem am Schrank 1 befestigten Röntgenröhrengehäuse 2, in dem eine Röntgenröhre 3 angeordnet ist. An dem Gehäuse 2 ist eine nach unten ragende Säule 4 befestigt, an deren freiem Ende eine Aufnahmeplatte 5 für eine nicht dargestellte Filmkassette angebracht ist. Auf der Säule 4 ist ein Längsschlitz 6 vorhanden, aus dem ein Zapfen 7 herausragt, an dem eine Kompressionsplatte 8 so befestigt werden kann, daß sie parallel zur Aufnahmeplatte 5 liegt.Zwischen der Aufnahmeplatte 5 und der Kompressionsplatte 8 ist eine Brustauflageplatte 9 aus Kohlefibermaterial angeordnet. Die Kompressionsplatte 8 ist motorisch entlang dem Schlitz 6 und relativ zur Brustauflageplatte 9 zur Einstellung ihres Abstandes von der Brustauflageplatte 9 einstellbar.

Ferner ist an dem Röntgenröhrengehäuse 2 ein weiteres, nach unten ragendes Lichtvisiergehäuse 10 befestigt, in dem ein später näher beschriebenes Lichtvisier angebracht ist, dessen zur Kompressionsplatte 8 gerichtetes Lichtbündel durch Stellmittel einstellbar ist. An dem Lichtvisiergehäuse 10 ist ein abnehmbarer Tubus 11 zur Strahleneinblendung angeschlossen. Das Röntgenröhrengehäuse 2 mit den Teilen 3 bis 11 kann zur Einstellung in bezug auf die Größe der Patientin gegenüber dem auf dem Boden stehenden Schrank 1 in der Höhe eingestellt werden.

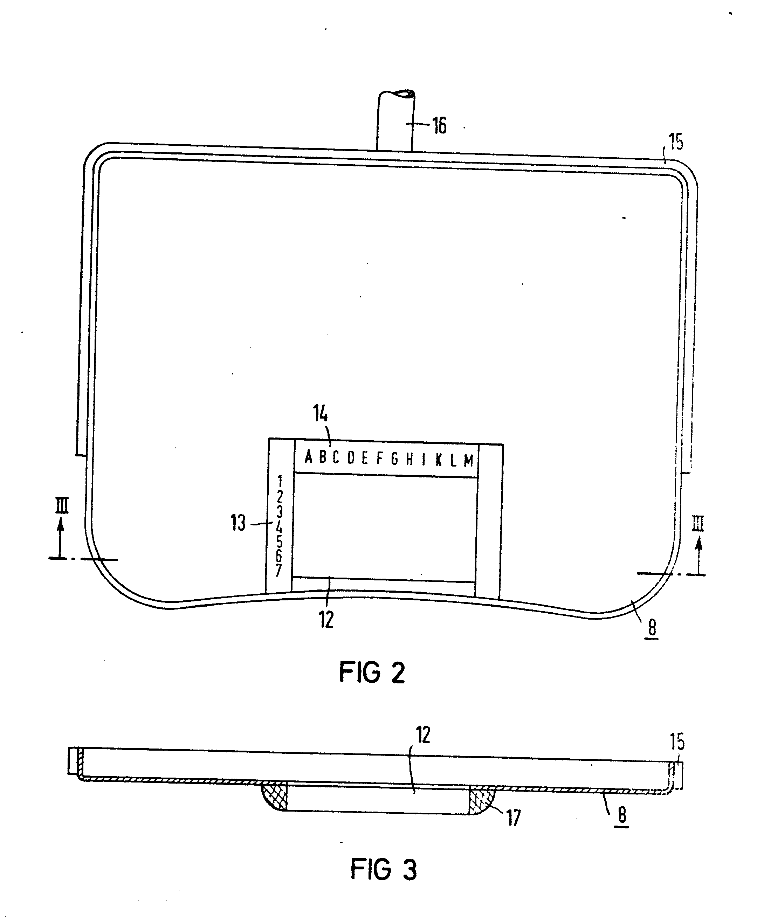

Die Fig. 2 zeigt die Kompressionsplatte 8 mit einem rechteckigen Ausschnitt 12, der am Rand der Kompressionsplatte und in der Mitte der der Patientin zugewandten Seite liegt. Der Ausschnitt 12 weist eine solche Größe auf, daß ein größerer Teil der Brust freigelassen wird und hat vorzugsweise eine Größe von etwa 40 x 65 mm. Am Rand des Ausschnitts sind strahlenundurchlässige oder strahlendämpfende Skalen 13, 14 angeordnet, die ein rechtwinkeliges Koordinatensystem bilden und die zum Aufsuchen eines bestimmten Punktes im Ausschnitt 12 dienen. Die Skala 14 weist Buchstaben und die Skala 13 Ziffern auf. Die Kompressionsplatte 8 ist auf einem Metallbügel 15 gehaltert, der über ein Rohrstück 16 mit dem Zapfen 7 verbindbar ist.

Die Fig. 3 zeigt, daß die Kompressionsplatte 8 im Randbereich ihres Ausschnitts 12 auf ihrer der Brustauflageplatte 9 zugewandten Seite in ihrer Dicke verstärkt
ist. Die Verstärkung ist vorzugsweise von einem in den
Ecken abgerundeten Holzrahmen 17 gebildet, um Sekundärstrahlung aufgrund der Verstärkung zu vermeiden. Die
Verstärkung beträgt ca. 1,5 cm. Der Holzrahmen 17 bewirkt eine optimale lokale Kompression, wenn er auf
die Brust gedrückt wird und spannt dadurch die Haut.
Die Reibung zwischen der Brust und der Kompressionsplatte 8 ist dabei so groß, daß die Brust sich während
der Untersuchung nicht verschiebt.

Die Fig. 4 zeigt das Lichtvisiergehäuse 10, das an dem
Röntgenröhrengehäuse 2 befestigt ist. In dem Gehäuse 10
sind zwei Lichtvisiere 22, 23 angeordnet, die mittels
nicht dargestellter Führungsmittel entlang von Schlitzen 18, 19 durch Stellknöpfe 20, 21 in dem durch die
Skalen 13, 14 gebildeten Koordinatensystem parallaxenfrei einstellbar sind. Jedes Lichtvisier 22, 23 weist
eine Lichtquelle 24, 25, eine Linsenoptik 26, 27 und
eine Öffnung zur Bildung von Lichtstrichen 28, 29 auf.
Durch Verschieben des Lichtvisiers 23 mittels des Stellknopfes 21 in dem Schlitz 19 ist der Strich 29 entlang
der Ziffernkoordinaten der Skala 13 (Pfeil 30) einstellbar. Durch Verschieben des Lichtvisiers 22 mittels
des Stellknopfes 20 in dem Schlitz 18 ist der Strich
28 entlang der Buchstabenkoordinaten der Skala 14
(Pfeile 31) einstellbar.

Bei der Untersuchung wird nach einer Übersichtsaufnahme mit einer Kompressionsplatte ohne Aussparung
die Brust der Patientin zwischen der Brustauflageplatte 9 und der Kompressionsplatte 8 eingepreßt und anhand des ersten Röntgenbildes so gelegt, daß der zu untersuchende Tumor in dem Ausschnitt 12 der Kompressionsplatte 8 liegt. Danach wird eine Röntgenaufnahme gemacht und der Film entwickelt, während die Brust der Patientin eingepreßt bleibt. Die Filmkassette, die zwischen der Auflageplatte 5 und der Brustauflageplatte 9 liegt, kann zur Filmentwicklung seitlich herausgezogen werden, ohne daß die Lage der Brust verändert werden muß. Auf dem entwickelten Film ist nun der Tumor gut zu sehen, da dieser Teil der Brust in der Aussparung 12 der Platte 8 liegt. Auf dem Film sind auch die Skalen 13, 14 deutlich zu sehen. Der Arzt mißt nun mit einem Maßstab denjenigen Koordinatenpunkt, in dem der Tumor liegt. Dann wird in dem Ausschnitt 12 mittels Lichtstriche 28, 29 der einstellbaren Lichtvisiere 22, 23 gemäß Fig. 4 der entsprechende Koordinatenpunkt 32 auf der Brust exakt eingestellt. Zur Vereinfachung der Einstellung können auch Skalen auf dem Lichtvisiergehäuse 10 entlang der Schlitze 18, 19 angeordnet sein, die den Skalen 13, 14 auf der Kompressionsplatte entsprechen. Bevor der Arzt mit der Biopsienadel in dem Koordinatenpunkt 32 die Brust punktiert, um eine Gewebeprobe zu entnehmen, kann der Tubus 11 entfernt werden, damit genügend Platz für die Biopsienadel vorhanden ist. In der Fig. 4 ist der Tubus 11 entfernt.

Patentansprüche

1. Röntgenuntersuchungsgerät für die weibliche Brust, mit einer von einem Röntgengenerator gespeisten Röntgenstrahlenquelle und einer Brustauflageplatte, an der eine Röntgenfilmkassette halterbar ist, bei dem zwischen der Röntgenstrahlenquelle und der Brustauflageplatte eine Kompressionsplatte parallel zur Brustauflageplatte gehaltert ist, die relativ zur Brustauflageplatte zur Einstellung ihres Abstandes von der Brustauflageplatte einstellbar ist, d a d u r c h g e k e n n z e i c h n e t , daß die Kompressionsplatte (8) einen Ausschnitt (12) solcher Größe aufweist, daß ein größerer Teil der Brust freigelassen wird und daß am Rand des Ausschnitts eine auf einem Röntgenfilm abbildbare Markierung (13, 14) zum Aufsuchen eines bestimmten Punktes im Ausschnitt (12) angeordnet ist.

2. Röntgenuntersuchungsgerät nach Anspruch 1, d a - d u r c h g e k e n n z e i c h n e t , daß der Ausschnitt (12) rechteckig ist und die Markierung (13, 14) ein rechtwinkeliges Koordinatensystem bildet.

3. Röntgenuntersuchungsgerät nach Anspruch 1 oder 2, d a d u r c h g e k e n n z e i c h n e t , daß die Kompressionsplatte (8) im Randbereich ihres Ausschnitts (12) auf ihrer der Brustauflageplatte (9) zugewandten Seite in ihrer Dicke verstärkt ist.

4. Röntgenuntersuchungsgerät nach Anspruch 3, d a - d u r c h g e k e n n z e i c h n e t , daß die Verstärkung (17) etwa 1,5 cm beträgt.

0002244

5. Röntgenuntersuchungsgerät nach Anspruch 3 oder 4, d a d u r c h   g e k e n n z e i c h n e t , daß die Verstärkung (17) von einem Holzrahmen gebildet ist.

6. Röntgenuntersuchungsgerät nach einem der Ansprüche 1 bis 5, d a d u r c h   g e k e n n z e i c h n e t , daß am Röntgenröhrengehäuse (2) eine Lichtvisieranordnung (22, 23) angebracht ist, deren zur Kompressionsplatte (8) gerichtetes Lichtbündel durch Stellmittel (20, 21) einstellbar ist.

7. Röntgenuntersuchungsgerät nach Anspruch 6, d a d u r c h   g e k e n n z e i c h n e t , daß den Stellmitteln (20, 21) eine Skalenanordnung am Röntgenröhrengehäuse (2) zugeordnet ist, die der Markierung (13, 14) auf der Kompressionsplatte (8) entspricht.

8. Röntgenuntersuchungsgerät nach Anspruch 6 oder 7, d a d u r c h   g e k e n n z e i c h n e t , daß sich an das Lichtvisier (22, 23) ein abnehmbarer Tubus (11) zur Strahlungseinblendung anschließt.

9. Röntgenuntersuchungsgerät nach einem der Ansprüche 1 bis 8, d a d u r c h   g e k e n n z e i c h n e t , daß die Brustauflageplatte (9) aus Kohlefibermaterial besteht.

10. Röntgenuntersuchungsgerät nach einem der Ansprüche 1 bis 9, d a d u r c h   g e k e n n z e i c h n e t , daß der Ausschnitt (12) am Rand der Kompressionsplatte (8) in der Mitte an der der Patientin zugewandten Seite liegt.

0002244

11. Röntgenuntersuchungsgerät nach Anspruch 2 und 10, d a d u r c h   g e k e n n z e i c h n e t ,   daß der Ausschnitt (12) eine Größe von etwa 40 x 65 mm hat.

0002244
77 P 8911    1/2

FIG 1

FIG 4

**FIG 2**

**FIG 3**

0002244

Europäisches
Patentamt

EUROPÄISCHER RECHERCHENBERICHT

Nummer der Anmeldung

EP 78 10 1463

| Kategorie | EINSCHLÄGIGE DOKUMENTE | | KLASSIFIKATION DER ANMELDUNG (Int.Cl.²) |
|---|---|---|---|
| | Kennzeichnung des Dokuments mit Angabe, soweit erforderlich, der maßgeblichen Teile | betrifft Anspruch | |
| | DE - A - 2 501 436 (B. KRAMANN & J.A. FESER) <br><br> * Seite 3, Zeile 28 - Seite 6, Zeile 24; Seite 7, Zeile 6 - Seite 8, Zeile 18; Seite 9, Zeile 27 - Seite 12, Zeile 8; Abbildungen 1-4 * <br><br> --- | 1,2,8, 10 | A 61 B  6/00 <br> 6/08 |
| | BE - A - 811 497 (A. HACHERELLE) <br><br> * Seite 4, Zeile 4 - Seite 5, Zeile 9; Abbildungen * <br><br> --- | 3 | |
| | | | RECHERCHIERTE SACHGEBIETE (Int. Cl.²) |
| | FR - A - 1 528 656 (G. PELUSO) <br><br> * Seite 1, linke Spalte, Zeile 33 - rechte Spalte, Zeile 19; Abbildungen 1-3 * <br><br> --- | 1,2,11 | A 61 B  6/00 <br> 6/04 <br> 6/08 <br> G 03 B 41/16 |
| | DE - B - 1 047 376 (A. MAIER) <br><br> * Spalte 3, Zeile 49 - Spalte 4, Zeile 29; Abbildung 3 * <br><br> --- | 1,6,8 | |
| | DE - C - 896 700 (O. SANDSTROEM) <br><br> * Seite 2, Zeilen 40-123; Abbildungen 1,4,5 * <br><br> --- | 1,2 | |
| | | | KATEGORIE DER GENANNTEN DOKUMENTE |
| | GB - A - 1 458 196 (R.B.W. LOWNDES) <br><br> * Seite 1, Zeilen 20-78; Seite 2, Zeilen 4-35; Abbildungen 1,2 * <br><br> --- <br><br> ./. | 1,2 | X: von besonderer Bedeutung <br> A: technologischer Hintergrund <br> O: nichtschriftliche Offenbarung <br> P: Zwischenliteratur <br> T: der Erfindung zugrunde liegende Theorien oder Grundsätze <br> E: kollidierende Anmeldung <br> D: in der Anmeldung angeführtes Dokument <br> L: aus andern Gründen angeführtes Dokument <br> &: Mitglied der gleichen Patent- familie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 23-02-1979 | RIEB |

EPA form 1503.1  06.78

**Europäisches Patentamt**

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int.Cl.²) |
|---|---|---|---|
| P | <u>DE - A - 2 640 248</u> (SIEMENS AG)<br>* Seite 6, Zeilen 1-26; Seite 7, Zeile 20 - Seite 10, Zeile 9; Abbildungen 1,2 * | 1,6 | |

RECHERCHIERTE SACHGEBIETE (Int.Cl.²)